# EUROPEAN PATENT SPECIFICATION

(11) **EP 0 725 142 B1**
(45) Date of publication and mention of the grant of the patent: **10.09.2003**
(21) Application number: 95922746.3
(22) Date of filing: 23.06.1995
(51) Int. Cl.: C12P 13/04, C12N 1/15

(54) **PROCESS FOR PRODUCING D-AMINO ACID BY USING COMPOSITE IMMOBILIZED ENZYME PREPARATION**
VERFAHREN ZUR HERSTELLUNG VON D-AMINOSÄURE DURCH EINE ZUSAMMENGESETZTE IMMOBILISIERTE ENZYMENVERBINDUNG
PROCEDE DE PRODUCTION DE D-AMINO ACIDE AU MOYEN D'UNE PREPARATION A BASE D'UNE ENZYME COMPOSITE IMMOBILISEE

(30) Priority: 24.06.1994 JP 14297794
(43) Date of publication of application: 07.08.1996
(73) Proprietor: KANEGAFUCHI KAGAKU KOGYO KABUSHIKI KAISHA, Kita-ku, Osaka-shi, Osaka 530 (JP)
(72) Inventor: NANBA, Hirokazu, Kakogawa-shi, Hyogo 675 (JP); YAMADA, Yukio, Kakogawa-shi, Hyogo 675-03 (JP); YAJIMA, Kazuyoshi, Akashi-shi, Hyogo 673 (JP); TAKANO, Masayuki, Kakogawa-shi Hyogo 675 (JP); IKENAKA, Yasuhiro, Akashi-shi, Hyogo 673 (JP); TAKAHASHI, Satomi, Kobe-shi, Hyogo 655 (JP); OHASHI, Takchisa, Kobe-shi Hyogo 657 (JP)
(74) Representative: Polz, Leo, Dr.
(86) International application number: JP9501257
(87) International publication number: WO96000296

(56) References cited:
- EP-A- 0 261 836
- WO-A-93/21336
- WO-A-94/00577
- GB-A- 1 587 116
- JP-A- 2 119 796
- JP-A- 2 276 586
- US-A- 4 211 840

## Description

### Field of the Invention

The present invention relates to a composite immobilized enzyme preparation and a process for producing D-amino acids using the preparation. In particular, the composite immobilized enzyme preparation of the present invention is useful for the production of D-α-amino acids which are intermediate compounds for the production of antibiotics, such as D-(p-hydroxyphenyl)glycine to be used for the production of the antibiotic, amoxycillin and the like.

### Backgrounds of the Invention

Optically-active D-amino acids are important compounds as intermediate compounds for drugs and it has been known that they can be produced efficiently by combining an asymmetric hydrolysis reaction of 5-substituted hydantoins into the corresponding D-N-carbamyl-α-amino acids with the enzymes, hydantoinases (hereinafter sometimes abbreviated as "Hase") (GB-A-2 042 531 and JP-B 62-30785), and an conversion reaction of the resultant D-N-carbamyl-α-amino acids into the corresponding D-α-amino acids with the enzymes, D-N-carbamyl-α-amino acid amidohydrolases (hereinafter sometimes abbreviated as "decarbamylase" or "DCase") (PCT/JP91/0696: WO 92/10579).

In addition, and the like disclose that the respective reactions are carried out more efficiently by using these enzymes in the form of so-called immobilized enzymes wherein they are immobilized on supports such as ion exchange resins and the like.

However, a two-step reaction has been employed for carrying out these reactions because the optimal and stable pH's of both enzymes are considerably different from each other. Therefore, both immobilized enzymes should be prepared separately and complicated reaction operations are required.

WO 93 21 335 A relates to a method producing D-amino acids from a racemic mixture using microorganisms containing hydantoinase and carbamylase enzymatic activities immobilized on a support. The individual enzymes hydantoinase and carbamylase have not been separated. Thus, according to this document, the microorganism is immobilized on the support not the enzyme.

US-A-4,211,840 and JP- 211 9796 A describe a method for producing D-α-amino acids by contacting a 5-substituted hydantoin with an enzyme capable of converting the 5-substituted hydantoin to the D-α-amino acid. Only one enzyme necessary for the conversion of 5-substituted hydantoin to the D-α-amino acid is used.

WO 94 00577 A relates to the production of a carbamoylase enzyme by expressing recombinant DNA encoding a carbamoylase gene in a homologous host. It also relates to specific recombinant DNA vectors comprising a carbamoylase enzyme and/or a hydantoinase enzyme to produce D-α-amino acids. By using the DNA described in this document D,L-5-substituted hydantoin is converted into its D-α-amino acid. However, WO 92 00577 A does not describe the immobilization of both enzymes, which can for example be obtained from one microorganism producing both by using DNA vectors, on a support.

EP-261 836 relates to an immobilized enzyme preparation. The enzyme preparation comprises an immobilized hydantoinase for the preparation of D(-)(optionally substituted phenyl)glycine or a N-carbamoyl derivative thereof by hydrolysis of 5-(optionally substituted phenyl)hydantoin. It was found that the presence of a divalent metal ion in an immobilized enzyme system imparts stability to the preparation. The hydantoin enzyme is obtained from a microorganism.

JP 211 9796 A relates to a process for producing a L-homophenylalanine by using a microorganism possessing an L-specific hydantoinase consisting of a hydantoin.hydrolase and N-carbamylamino acid hydrolase, or an enzyme of this type is allowed to act on 5-benzyl methyl hydantoin so as to produce L-homophenylalanine.

JP 227 6586 A relates to a process for producing D-homophenylalanine. D-homophenylalanine is produced by a microorganism belonging to Pseudomonas, Achromobacter, Alcaligenes, Serratia, Aspergillus and Rhizomocur, or a D-hydantoin-decomposable enzyme derived from any of these microorganisms, which is allowed to act on 5-benzylmethyl hydantoin.

### Objects of the Invention

The present invention relates to a technique for producing D-α-amino acids efficiently by using an immobilized enzyme resin obtained by immobilizing simultaneously both hydantoinase and decarbamylase directly on one immobilizing resin support in a coexisting state of the enzymes (hereinafter referred to as a "composite enzyme").

In these two enzymatic reactions for converting 5-substituted hydantoins into D-α-amino acids, in general, the optimal pH of the hydantoinase reaction is pH 8 to 9 and the solubility of the substrate is increased as increase in pH. In addition, the racemic reaction of the hydantoin ring is promoted in an alkaline range. Therefore, it is desired to carry out the hydantoinase reaction in the pH ranging from 7 to 10, preferably in an alkaline range. On the other hand, in general, the optimal pH of the decarbamylase reaction is pH 6.5 to 9.0 but the hindrance of the reaction by ammonia formed is remarkably increased as increase in pH. Therefore, it is desired to carry out the decarbamylase reaction at pH about neutrality.

If so-called one-step reaction can be employed for carrying out these reactions, i.e., these two enzymatic reactions can be carried out in one reaction vessel simultaneously, in comparison with so-called two-step reaction wherein two different enzymes react with the substrates separately, reaction operations become simple and the overall reaction time can be shortened. In addition, by combining the hydantoinase reaction which is a reversible reaction and the decarbamylase reaction which is a irreversible reaction, the conversion yield of hydantoin can be improved as well as the subsequent purification of D-amino acids can be simplified. Thus, it is expected to significantly reduce the production cost. However, when the respective enzymes are immobilized on different immobilizing supports and they are mixed upon using them, the reactivities become inferior because of the difference in optimal pH and there is a problem in the stability of the immobilized enzymes.

The present inventors have intensively studied to produce a composite immobilized enzyme preparation wherein both enzymes are simultaneously immobilized directly on one immobilizing support.

If these two enzymes are immobilized simultaneously, two enzymatic reactions occur successively in micro-spaces of resins. Therefore, movement of the substrate for a decarbamylase, a D-N-carbamyl-α-amino acid, from immobilized Hase to immobilized DCase by diffusion is not required and pH variation in the micro-spaces can be minimized. Thus it is expected that, in addition to increase in reactivitie of the respective enzymes and relief of the product hindrance due to ammonia, stability of enzymes upon using repeatedly is improved.

### Summary of the Invention

The present invention provides a process for the efficient production of a D-amino acid from the corresponding DL-5-substituted hydantoin by one-step reaction which comprises using a composite immobilized enzyme at a pH about neutrality, said composite immobilized enzyme being obtained by simultaneous immobilization of a mixture of a hydantoinase having its optimal pH within an alkaline range and a D-N-carbamyl-α-amino acid amidohydrolase having its optimal pH about neutrality in a coexisting state diretly on an immobilizing support of an anionic ion exchange resin. (hereinafter referred to as composite enzyme).

### Detailed Explanation of the Invention

As for the hydantoinase used in the present invention, the enzymes from animals, plants and microorganisms can be used. The hydantoinases from microorganisms are suitable for industrial production. As such a microorganism, those disclosed in GB-A-2 042 531 (JP-B-62-30758) are exemplified. The bacteria include Achromobacter, Aerobacter, Aeromonas, Agrobacterium, Alcaligenes, Arthrobacter, Bacillus, Brevibacterium, Corynebacterium, Enterobacter, Erwinia, Escherichia, Klebsiella, Microbacterium, Micrococcus, Protaminobacter, Proteus, Pseudomonas, Sarcina, Serratia, Xanthomonas and the like. The Actinomycetes include Actinomyces, Mycobacterium, Nocardia, Streptomyces, Actinoplanes and the like. The filamentous fungi include Aspergillus, Paecilomyces, and Penicillium and the like. The yeasts include Candida, Pichia, Rhodotorula, Torulopsis and the like.

Among the above microorganisms, examples of strains which have relatively high hydantoinase activities and are excellent in practical use include Aerobacter cloacae IAM 1221, Agrobacterium rhizogenes IFO 13259, Brevibacterium incertum IFO 12145, Corynebacterium sepedonicum IFO 3306, Microbacterium flavum ATCC 10340, Micrococcus roseus IFO 3764, Pseudomonas striata IFO 12996, Mycobacterium smegmatis ATCC 607, Nocardia corallina IFO 3338, Streptomyces flaveolus IFO 3241, Bacillus sp. KNK108 (PERM P-6056), Bacillus sp. KNK245 (FERM BP-4863) and the like.

In addition, the enzymes produced by artificial microorganisms in which hydantoinase productivity is imparted by gene recombinant technique, for example, E. coli HB101pTH104 (FERM BP-4864), or increased or the dihydropyrimidinase having similar activity as disclosed in JP-A 53-136583 can be used.

As for the decarbamylase used in the present invention, the origins thereof is not specifically limited and those derived from animals, plants and microorganism can be used. However, for industrial production, the enzymes from microorganisms are suitable. Examples of such microorganisms include naturally occurring microorganisms such as Agrobacterium Pseudomonas, Arthrobacter, Alcaligenes, Achromobacter, Moraxella, Paracoccus, Aerobacter, Aeromonas, Brevibacterium, Bacillus, Flavobacterium and Serratia disclosed in JP-B 57-18793, GB-A-2 022 581 (JP-B-63-20520) and GB-A-2 042 531 (JP-B-1-48758), or the artificial microorganisms described in WO 92/10579 in which decarbamylase productivity is imparted or increased by gene recombinant technique. Representative examples of such microorganisms include Agrobacterium sp. KNK712 (FERM BP-1900), Pseudomonas sp. KNK003A (FERM BP-3181), Pseudomonas sp. KNK505 (FERM BP-3182), Escherichia coli JM109pAD108 (FERM BP-3184), E. coli JM109pPD304 (FERM BP-3183) and the like.

When a stabilized decarbamylase in which the amino acid responsible for heat resistance of the decarbamylase is replaced by another one is used, the following transformants disclosed in WO 94/03613 can be used. For example, the transformants include E. coli JM109pAD402 (FERM BP-3912), E. coli JM109pAD404 (FERM BP-3913), E. coli JM109pAD406 (FERM BP-3914), E. coli JM109pAD416 (FERM BP-3915), E. coli JM109pAD429 (FERM BP-4035), E. coli JM109pAD421, E. coli JM109pAD422, E. coli JM109pAD423, E. coli JM109pAD424 (FERM BP-4034), E. coli JM109pAD426, E. coli JM109pAD427, E. coli JM109pAD451, E. coli JM109pAD455 (FERM BP-4036), E. coli HB101pNT4553 (FERM BP-4368) or the like. When such stabilized enzymes are used, better results can be obtained in repeated use of the composite immobilized enzyme preparation of the present invention.

The enzymes used in the present invention can be produced simultaneously by using a microorganism which is capable of producing both enzymes. Alternatively, the enzymes can be produced separately or simultaneously using microorganisms which are capable of producing respective enzymes alone. As the microorganism which is capable of producing both enzymes, a microorganism isolated from a natural source such as an Agrobacterium disclosed in GB-A-2 022 581 (JP-B-63-20520) and the like can be used. In addition, a recombinant microorganism prepared by isolating genes of both enzymes from the microorganisms isolated from a natural source and introducing them into a host, for example, the microorganisms disclosed in WO 94/00577, can be used. A microorganism which produces both enzymes simultaneously is also Agrobacterium sp KNK-712 (FERM BP-1900), Rhizobium sp. KNK1415 (FERM BP-4419) or Pseudomonas sp. KNK003A (FERM BP-3181). Alternatively, genes of both enzymes can be isolated from the same or different microorganisms and inserted into the same vector in the expressible form of both genes, or inserted into different vectors having different replication modes, for example, pUC19 and pACYC184. Then, the recipient host such as E. coli can be transformed with the above vector or vectors so that a single microorganism can produce both enzymes. In these cases, by selecting the kinds of promoters having various capabilities and the plasmids having different copy numbers, the ratio of production of each enzyme can be varied according to a particular purpose. However, it is preferred to adjust the ratio so as to obtain almost equal amounts of enzyme proteins. In the case that microorganisms which produce different enzymes separately are used, strains isolated from natural sources or recombinant microorganisms prepared by using gene recombinant technique can also be used. In such case, the production of the enzymes can be carried out either by cultivating the producer microorganisms separately, or cultivating in a mixed culture at various ratios, preferably, at such a ratio that almost equal amounts of the enzymes can be produced.

The cultivation can be carried out aerobically, for example, by shaking culture using flasks or by spinner culture with aeration. As for culture medium used in the cultivation, normally, an nutrient medium which contains generally used natural nutrients such as meat extract and polypeptone and the like can be used. When hydantoinase is produced separately or simultaneously with decarbamylase, a good result can be obtained by carrying out the cultivation with addition of manganese ion in an amount of, for example, as manganese chloride, 1 to 100 mg/liter, preferably about 20 mg/liter. And, in so far as the enzymes are under the conditions that the enzymatic activities can be exhibited the enzymes can exist in any form, and can be accompanied by any substance.

In the preparation or use of the composite immobilized enzyme preparation of the present invention, better results can be obtained by adding an antioxidant for a repeated use. As such an antioxidant, there can be used dithiothreitol, 2-mercaptoethanol, L-cystein hydrochloride, cysteamine hydrochloride, dithioerythritol, a mixture of dithiothreitol and dithioerythritol, reduced glutathione and the like.

An immobilized support used can be varied according to particular use conditions of the immobilized enzymes. When a crude enzyme solution such as a cell-free extract, or a partially purified enzyme solution treated by ammonium sulfate precipitation or the like are subjected to immobilization, polymer supports having ion exchanging groups or covalent bonding groups can be used.

As for a polymer support having an ion exchanging group, for example, Duolite (registered trade mark) series, or Amberlite IRA (registered trade mark) series, the exchanging groups of which are primary, secondary, tertiary and quaternary amines; or a polystyrene resin having a diethanol type functional group, for example, Diaion EX can be used.

As for a support with a covalent bonding group, a substituted polymethacrylate polymer having an aldehyde as a bonding group, a high density alumina covered with a complex of polyethyleneimine/glutaraldehyde and the like can be used.

The composite immobilized enzyme preparation of the present invention is produced as follows. A solution of the crude composite enzyme in which activities of Hase and DCase are appropriately adjusted is contacted with a support to adsorb the respective enzymes and treated with a cross-linking agent for stabilization. To prepare a solution of the crude composite enzyme, firstly, each enzyme is produced by cultivating microbial cells. In this case, a cell-free extract can be prepared by collecting the cells and disrupting them with, for example, sonication, mechanical disruption (for example, homogenizer) or enzyme treatment, when a microorganism capable of producing the enzymes simultaneously is used. When a solution of the composite enzyme is prepared by using different microorganisms, it can be prepared by cultivating the microorganisms separately, collecting the cells, preparing respective cell-free extracts and mixing them. Or, it can be prepared by cultivating respective producer microorganisms together and disrupting the cells at the same time to prepare a cell-free extract in which both two enzymes are mixed. When both enzymes are produced by different microorganisms, the productivities vary to a large extent according to the kinds of the microorganisms and difference in the cultivation methods. However, in general, regarding hydantoinase, a normal producer microorganism produces hydantoinase at about 0.1 to about 10 units/ml (one unit of the enzyme used herein is defined as an amount required to convert the substrate 5-(p-hydroxyphenyl)hydantoin into D-N-carbamyl-p-hydroxy-phenylglycine at pH 8.7, 40°C, for 1 min) and a recombinant artificial producer microorganism produces it at about 5 to about 150 units/ml. Regarding decarbamylase, a normal producer microorganism produces decarbamylase at about 0.01 to about 2 units/ml (one unit of the enzyme used herein is defined as an amount required to convert the substrate D-N-carbamyl-p-hydroxyphenylglycine into D-p-hydroxyphenylglycine at pH 7.0, 40°C, for 1 min) and a recombinant artificial producer microorganism produces it at about 0.1 to about 20 units/ml. The ratios of Hase and DCase activities in a solution of the crude composite enzyme are adjusted so that the reaction producing D-α-amino acid from 5-substituted hydantoin can proceed most efficiently. As described hereinafter, this reaction is carried out at a pH of neutrality which is near the optimal pH for DCase and therefore the conditions are different from those for exhibiting maximal activity of Hase. Then, it is desired that both enzymatic activities are adjusted so that almost equal activities are exhibited at the reaction pH. For example, in the case of carrying out the reaction at pH 7.5, the desired composite immobilized enzyme preparation can be produced by carrying out the immobilization reaction using an enzyme solution in which there are almost equal amounts of proteins and enzymatic activities so that hydantoinase is about five times as much as the DCase in units. To maintain such activities after immobilization, it is desired that the ratio of the enzyme activities (Hase : DCase) in a solution of the crude composite enzyme should be in a range of 1 to 10 : 1. Therefore, after disruption of the microbial cells and before adsorption, the activities of the crude composite enzyme are adjusted to such levels.

In addition, to adsorb appropriate amounts of enzymes on the support, it is preferred to adjust the concentration of decarbamylase in the solution of the crude composite enzyme to 10 to 300 units/ml. The activity adsorbed is about 20 to about 90% of the activity added and no substantial difference can be seen between both enzymes.

The support is used after activation of its exchanging group with, for example, aqueous solution of sodium chloride and equilibrating, for example, in a buffer solution. It is preferred that the ratio of the solution of the crude composite enzyme and the support is adjusted so that the total amount of the protein in the crude enzyme solution are almost the same as the maximum adsorption capacity of the support. If necessary, 1 to 10 mM of an antioxidant and/or 0.5 to 20 mM of manganese ion can be present. The mixture is stirred at 4 to 30°C, preferably at 15°C, and the support is collected by filtration after the amount of enzyme adsorbed reaches the given amount (normally 8 to 48 hours, preferably this is carried out under the atmosphere of inert gas, such as nitrogen). Then, the support is washed and insolubilized by treating with a cross-linking agent to stabilize it. As for a cross-linking agent, a known agent such as at less than 1%, preferably 0.1 to 0.2% glutaraldehyde can be used. The crosslinked composite enzyme immobilized preparation is washed with distilled water and a buffer solution (preferably containing 0.1 to 20 mM, normally 1 to 5 mM antioxidant as described above) and stored in wet state in a sealed vessel at a low temperature (4°C). In general, the activities of the composite immobilized enzyme preparation thus obtained are 5 to 80 units/g support with respect to decarbamylase. For hydantoinase, its activity is 1 to 10 times as much as the decarbamylase activity according to the adsorption conditions.

The process for producing D-α-amino acids from 5-substituted hydantoins by using the composite immobilized enzyme preparation of the present invention will be described hereinafter.

The reaction is carried out by reacting the substrate, 5-substituted hydantoin, with the composite immobilized enzyme preparation, if necessary, in the presence of an antioxidant agent and/or manganese ion. Normally, it is preferred that the reaction is carried out in the presence of 0.1 to 20 mM of an antioxidant agent and manganese ion. The reaction proceeds according to the following reaction scheme: 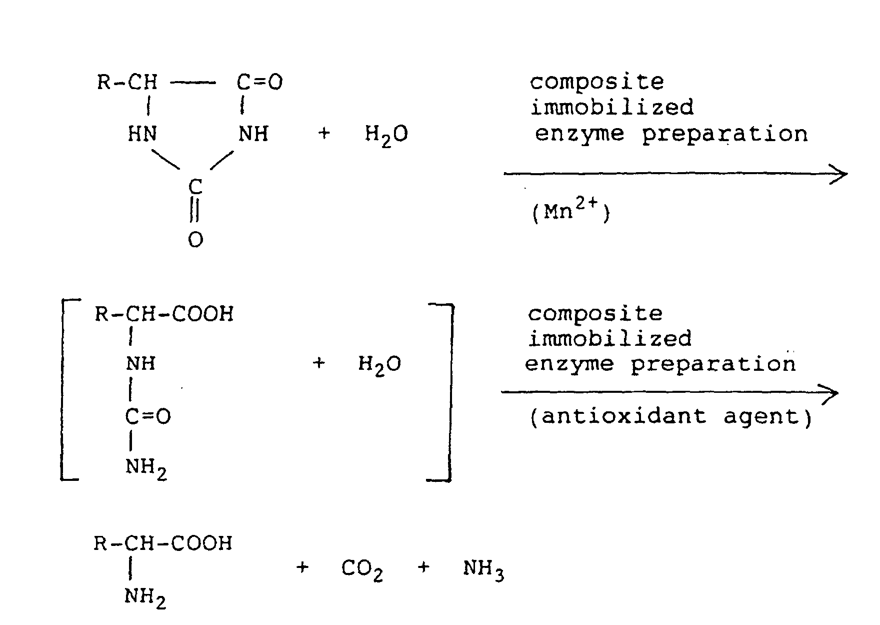 wherein R represents phenyl group, phenyl group substituted with hydroxy group, alkyl group, substituted alkyl group, aralkyl group or thienyl group.

The concentration of the substrate 5-substituted hydantoin to be used is 0.1 to 30% (w/v), preferably 1 to 5% (w/v). It is preferred that the amount of the composite immobilized enzyme preparation to be used is about 10 to about 20 units/g substrate as decarbamylase activity. The reaction temperature varies according to particular enzymes but, in general, it is 30 to 60°C. Enzymes having heat resistance be used at much higher temperature.

The reaction pH is suitably selected from the range of 6.5 to 8.0. The pH range is almost optimal for DCase. However, it is considerably far from the optimal pH for Hase and the Hase activity decreases several times as low as the activity at the optimal pH. The rate of racemization also decreases. However, ultimately, the production of D-α-amino acid is governed by the enzyme of the last step, DCase, it is preferred to set the amount of the enzyme and the reaction conditions based on the DCase (i.e., the optimal conditions for DCase) and to combine Hase activity in proportion to that DCase activity. In general, the ratio of the enzymes to be adsorbed to the support is adjusted so that almost equal activities can be expressed, although this varies according to particular reaction conditions. The "almost equal activities" mean the situation where the ratio of the hydantoinase activity measured at pH 7.5 (represented in "units (pH 7.5)"), and the decarbamylase activity as described above is about 0.5 to 1.5 : 1. The reaction is carried out with adjusting a reaction pH to the pH range thus selected, normally to pH 7.5. By these operations, the unfavorable conditions for Hase are overcome and the reaction equilibrium is declined toward the production of D-N-carbamyl amino acid. Thus, the reaction can be carried out more efficiently than expected and the conversion of the substrate, a 5-substituted hydantoin, can be improved. When the reaction is carried out at an alkaline pH range near the optimal pH of hydantoinase, the decarbamylase activity decreases to below the half of the activity at the optimal pH, though the production of a D-N-carbamyl amino acid can proceed successfully. In addition, it has been observed that the whole yield decreases and that the stability of decarbamylase itself is lowered because the reaction is largely inhibited by ammonia produced by the reaction. For these reasons, the reaction using the composite immobilized enzyme preparation can be carried out efficiently when the composite immobilized enzyme preparation having such immobilization ratio that the decarbamylase reaction is slightly rate-determining is used under conditions near the optimal reaction conditions and the substrate concentration of decarbamylase. In addition, it is advantageous that the whole activities of the composite immobilized enzyme preparation is easily controlled because the productivity of Hase is high and the DCase catalyzes the irreversible reaction.

The reaction is carried out normally by column method or by suspending the composite immobilized enzyme preparation in a reaction vessel. In the latter case, a batch reaction is usually carried out and the reaction time is about 6 to about 48 hours per batch. By using the composite immobilized enzyme preparation of the present invention, the corresponding D-amino acid can be produced from a 5-substituted hydantoin at a high yield in a one-step reaction. The following examples further illustrate the present invention in detail but are not to be construed to limit the scope thereof.

### Example 1

Firstly, for preparing an enzyme solution of hydantoinase, Bacillus sp. KNK108 (FERM P-6056) was inoculated to a 250 ml of seed culture medium (meat extract 1.0%, polypeptone 1.0%, yeast extract 0.5% (pH 7.0)) and cultivated at 33°C for about 25 hours. This seed culture was inoculated to 2.5 liter of a culture medium (meat extract 1.0%, polypeptone 1.0%, yeast extract 0.5%, uracil 0.1%, MnCl₂ 20 ppm (pH 7.5)) and cultivated at 33°C for about 16 hours. The microbial cells were collected by centrifugation and suspended in 50 ml of 20 mM MnSO₄ aqueous solution. After adjusting the pH to 8.5, the cells were disrupted by sonication and the residue was removed to obtain a crude enzyme solution of hydantoinase (107 units/ml).

In addition, to prepare an enzyme solution of decarbamylase, E. coli HB101pNT4553 (FERM BP-4368) was cultivated in 20 ml of 2YT medium (Bacto peptone 1.6%, Bacto yeast extract 1.0%, NaCl 0.5%) supplemented with 50 µg/ml ampicillin at 37°C for about 16 hours. This culture was inoculated to 1.4 liter of 2YT medium in an amount of 1%, and cultivated at 37°C for about 28 hours. The cells were collected by centrifugation and suspended to 140 ml of 5 mM dithiothreitol solution. After adjusting the pH to 7.0, the cells were disrupted by sonication and the residue was removed to obtain the supernatant as a crude enzyme solution of decarbamylase (36 units/ml).

### Example 2

By using the crude enzyme solutions obtained in Example 1, immobilization of the enzymes was carried out. Duolite A-568 (Rohm & Haas) as a immobilization support was washed with firstly 1M NaCl and ion exchanged water and then put into the ion exchanged water and the pH was adjusted to 7.5. To 8.4 g of this resin were added 20 ml of the crude enzyme solution of hydantoinase and 11.5 ml of the crude enzyme solution of decarbamylase the pH of both of which had been adjusted to 7.5 and the mixture was stirred at 15°C for about 20 hours under nitrogen atmosphere. After washing this resin twice with 0.5 mM MnSO₄ solution, the resin was suspended in five time volumes of ion exchanged water. After the pH was adjusted to 7.5, the suspension was stirred for 35 minutes with addition of 544 µl of 2.5% glutaraldehyde with portions. This suspension was treated with 50 mM Tris-HCl (pH 7.5), 5 mM DTT and 1 mM MnSO₄ overnight and then the composite immobilized enzyme preparation was collected by filtration (hydantoinase activity 8.2 units (pH 7.5) and decarbamylase activity 9.9 units per 1g resin).

Then, as controls, resins to which two enzymes were separately immobilized were prepared.

Each of 20 ml of the crude enzyme solution of hydantoinase and 60 ml of the crude enzyme solution of decarbamylase was mixed with each of 4.2 g and 22.0 g of the above immobilization support and the same operations as described above was carried out to obtain each immobilized enzyme resin in which each enzyme was separately immobilized (hydantoinase immobilized enzyme: 24 units/g resin, 2.7 units/g resin (pH7.5), and decarbamylase immobilized enzyme: 37 units/g resin).

### Example 3

By using the composite immobilized enzyme preparation and control immobilized enzymes containing respective single enzymes obtained in Example 2, the reactions to produce the corresponding D-α-amino acid from a 5-substituted hydantoin were carried out.

After addition of 1g of 5-(p-hydroxyphenyl)hydantoin as the substrate to 100 ml of 0.1 M KPB (pH 7.5), 1 mM MnSO₄ and 5 mM DTT, nitrogen gas was sufficiently bubbled in and the reaction conditions were adjusted to 40°C and pH 7.5. In order to obtain the same enzymatic activities of both enzymes, 0.97 g of the composite immobilized enzyme preparation or 3.0 g of the hydantoinase immobilized enzyme and 0.26 g of the dacarbamylase immobilized enzyme were added. The reaction was carried out with bubbling of nitrogen gas and controlling the reaction pH to 7.5 with 2N H₂SO₄ or 6N NaOH. The reaction was carried out for 29 hours with periodical samplings. The amount of p-hydroxyphenylglycine produced at each sampling point was determined by high performance liquid chromatography (Nippon Bunko, Finepack SIL C-18 column).

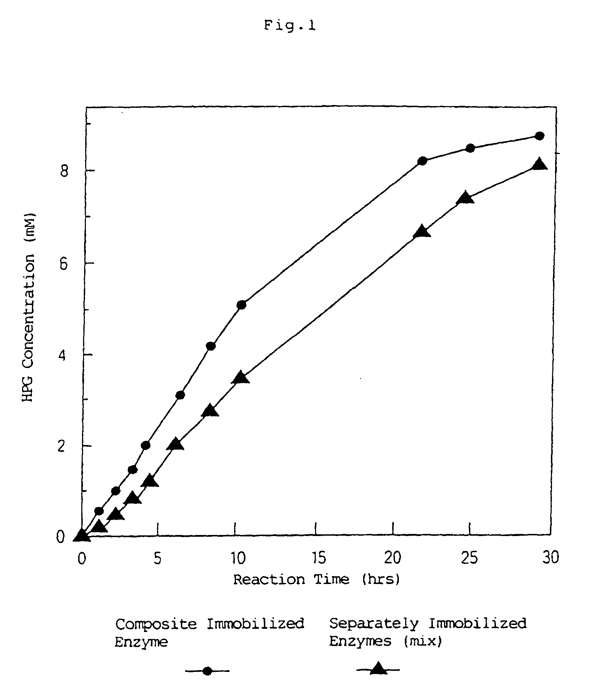

The results are shown in Fig. 1.

As is seen from Fig. 1, it is found that the reaction can be carried out more efficiently when two enzymes were immobilized simultaneously on the same resin.

### Example 4

The stability of the enzyme activities was investigated by carrying out the reaction repeatedly with the composite immobilized enzyme preparation. Two grams of 5-(p-hydroxyphenyl)hydantoin was added to 100 ml of 1 mM MnSO₄ and 5 mM DTT and pH was adjusted to 7.5. To the mixture was added 8.9 g of the composite immobilized enzyme preparation obtained in Example 2 was added and the reaction was carried out at 40°C for 23 hours with bubbling of nitrogen gas and controlling the pH to 7.5. After filtering the reaction mixture with suction, another mixture was added to the composite immobilized enzyme according to the same manner as described above and the reaction was carried out. This operation was repeated five times and both enzyme activities were determined at the end of each reaction.

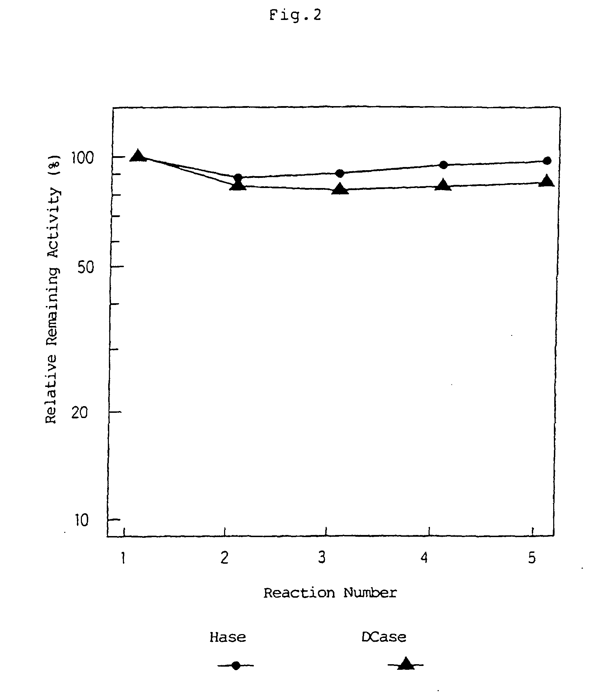

The relative activities to those in the first reaction are shown in Fig. 2.

The decrease in the activities were scarcely observed in these five reactions.

### Example 5

For preparing an enzyme solution of hydantoinase, E. coli HB101pTH104 (FERM BP-4864) containing a hydantoin gene from Bacillus sp. KNK245 (FERM BP-4863) was cultivated in 20 ml of 2YT medium at 37°C for about 16 hours. This culture was transferred to 1.2 liter of 2YT medium supplemented with 50 µg/ml of ampicillin and 400 ppm of MnCl₂·4H₂O and cultivated for 26 hours at 37°C. The cells were collected by centrifugation and suspended in 80 ml of 1 mM MnSO₄ aqueous solution. After adjusting the pH to 8.5 with ammonia water, the cells were disrupted by sonication and the residue was removed by centrifugation. After adjusting the pH of the supernatant to 8.5, heat treatment at 60°C was carried out for 20 min. The denatured proteins were removed by centrifugation to obtain a crude enzyme solution of hydantoinase (1,100 units/ml).

By using this crude enzyme solution and a crude enzyme solution of decarbamylase prepared according to the same manner as described in Example 1 (240 units/ml), the composite immobilized enzyme preparation was prepared according to the same manner as described in Example 2. By using 30 ml of the crude enzyme solution of decarbamylase and 13 ml of the crude enzyme solution of hydantoinase, the enzymes were immobilized on 29 g of the resin according to the same manner as described in Example 2 (decarbamylase: 45 units, and hydantoinase: 119 units, 44 units (pH 7.5) per 1 g of resin)

As controls, resins on which two enzymes were separately immobilized were used. The decarbamylase immobilized enzyme (43 units/g-resin) was prepared as described in Example 2. The hydantoinase immobilized enzyme was prepared according to the method described in Example 2 by mixing 21.8 g of the resin for immobilization with 60 ml of the crude enzyme solution (177 units/g-resin, 51 units/g (pH 7.5))

### Example 6

By using 5 g of the composite immobilized enzyme preparation obtained in Example 5, and as immobilized enzymes prepared by immobilizing respective enzymes on different resins, a mixture of 5.6 g of the hydantoinase immobilized enzyme and 5.2 g of the decarbamylase immobilized enzyme obtained in Example 5 (the decarbamylase activity (pH 7.5 ) was equal to the composite immobilized enzyme preparation and the hydantoinase activity (pH 8.7) was twice as much as the composite immobilized enzyme preparation), the reaction was carried out with 3% substrate according to the method described in Example 3.

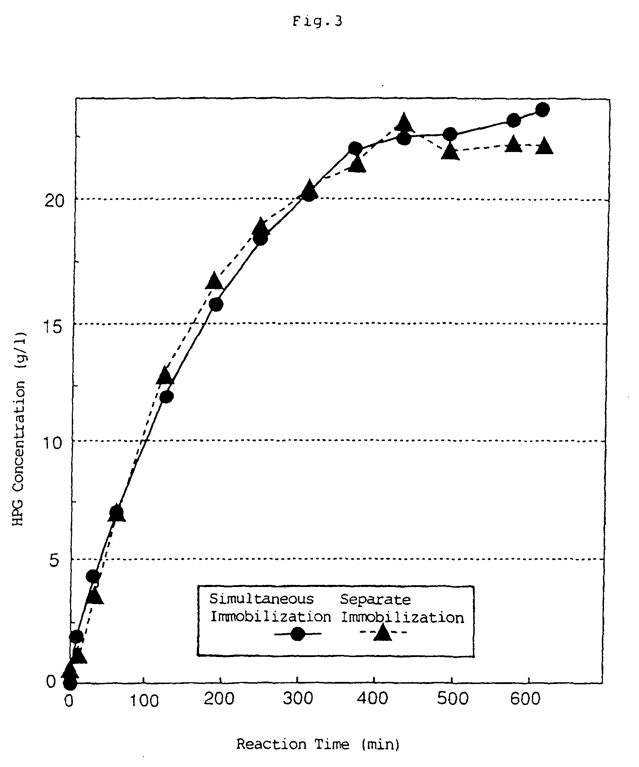

The results are shown in Fig. 3.

As shown in Fig. 3, the reactivity of the composite immobilized enzyme preparation was similar to the reactivity of the separately immobilized enzymes in spite that the hydantoinase activity was one half of the separately immobilized enzyme. Thus, it has been found that the reaction by the composite immobilized enzyme preparation is more efficient and that the amount of the expensive resin for immobilization can be largely reduced.

### Example 7

By using each 5 g of the composite immobilized enzyme obtained in Example 5, the effect of the reaction pH was investigated. In three pH levels of 7.0, 7.25 and 7.5, the reactions with 3% substrate were carried out. The times required to convert 99% of the substrate was measured. The times were 5.5, 5.4 and 6.75 hours, respectively. Thus, it have been found that pH 7.0 and 7.25 are advantageous to the reaction.

As described hereinabove, according to the present invention, by using of the composite immobilized enzyme preparation produced by immobilizing the hydantoinase and the decarbamylase simultaneously on the same resin in the production of the corresponding D-α-amino acids from 5-substituted hydantoins, it is possible to carry out the one-step reaction with much simpler operations than two-step reaction and more efficiently than the reaction by the mixture of the two immobilized enzymes obtained by immobilizing these enzymes separately to the different resins.

### Brief Explanation of the Drawings

Fig. 1 is a graph illustrating the time courses of the reactions for the production of the corresponding D-p-hydroxyphenylglycine from 5-(p-hydroxyphenyl)hydantoin in Example 3 by using the composite immobilized enzyme preparation of the present invention and the mixture prepared by immobilizing hydantoinase and decarbamylase separately to the different resins.
Fig. 2 is a graph illustrating the stability of the enzymes in repeated reactions by using the composite immobilized enzyme preparation in Example 4.
Fig. 3 is a graph illustrating the comparison of the activity of the composite immobilized enzyme preparation with the activity of the mixture of the separately prepared immobilized enzyme preparations (the hydantoinase activity is twice as much as the composite immobilized enzyme preparation) in Example 6.

## Claims

1. A process for the production of a D-amino acid from the corresponding DL-5-substituted hydantoin by one-step reaction which comprises using a composite immobilized enzyme at a pH about neutrality, said composite immobilized enzyme being obtained by simultaneous immobilization of a mixture of a hydantoinase having its optimal pH within an alkaline range (hereinafter abbreviated as Hase) and a D-N-carbamyl-α-amino acid amidohydrolase having its optimal pH about neutrality (hereinafter abbreviated as DCase) directly on an immobilizing support.

2. A process for the production of a D-amino acid according to claim 1, wherein a composite enzyme to be used for immobilization is prepared by producing the Hase and the DCase separately and then mixing them.

3. A process for the production of a D-amino acid according to claim 1 or 2, wherein the Hase to be used is that derived from a microorganism belonging to the genus Bacillus.

4. A process for the production of a D-amino acid according to claim 1 or 2, wherein the Hase to be used is that derived from Bacillus sp. KNK108 (FERM P-6056) or Bacillus sp. KNK245 (FERM BP-4863).

5. A process for the production of a D-amino acid according to claim 1 or 2, wherein the Hase to be used is that derived from a recombinant E. coli, particularly, E. coli HB101pTH104 (FERM BP-4864).

6. A process for the production of a D-amino acid according to claim 1 or 2, wherein the DCase to be used is that derived from Agrobacterium sp. KNK712 (FERM BP-1900), Pseudomonas sp. KNK003A (FERM BP-3181), Pseudomonas sp. KNK505 (FERM BP-3182), E. coli JM109pAD108 (FERM BP-3184), E. coli JM109pPD304 (FERM BP-3183).

7. A process for the production of a D-amino acid according to any of claims 1-4 or 6, wherein said DCase is that derived from a recombinant microorganism selected from the group consisting of *E.coli* JM109pAD402 (FERM BP-3912), *E.coli* JM109pAD404 (FERM BP-3913), *E.coli* JM109pAD406 (FERM BP-3914), *E.coli* JM109pAD416 (FERM BP-3915), E.coli JM109pAD429 (FERM BP-4035), *E.coli* JM109pAD421, *E. coli* JM109pAD422, *E.coli* JM109pAD423, E.coli JM109pAD424 (FERM BP-4034), *E.coli* JM109pAD426, *E.coli* JM109pAD427, *E.coli* JM109pAD451, *E.coli* JM109pAD455 (FERM BP-4036), or *E.coli* JM109pAD4553 (FERM BP-4368), the stability of which is improved by amino acid substitution of the DCase derived from *Agrobacterium sp*. KNK712 (FERM BP-1900).

8. A process for the production of a D-amino acid according to claim 1, wherein a composite enzyme used for immobilization is prepared by producing Hase and DCase simultaneously.

9. A process for the production of a D-amino acid according to claim 8, wherein a microorganism which produces the Hase and the DCase simultaneously is Agrobacterium sp. KNK-712 (FERM BP-1900), Rhizobium sp. KNK1415 (FERM BP-4419) or Pseudomonas sp. KNK003A (FERM BP-3181).

10. A process for the production of a D-amino acid according to claim 8, wherein the composite enzyme is produced by cultivating a host containing a plasmid prepared by ligating a Hase gene and a DCase gene to the same vector.

11. A process for the production of a D-amino acid according to claim 10, wherein the host containing a plasmid prepared by ligating the Hase gene and the DCase gene to the same vector is E. coli HB101pPHD301 (FERM BP-4866) or E. coli JM109pAHD101.

12. A process for the production of a D-amino acid according to claim 8, wherein the composite enzyme is produced by ligating the Hase gene and the DCase gene to different vectors having different modes of replication, introducing these vectors into the same host and cultivating the host.

13. A process for the production of a D-amino acid according to claim 8, wherein the composite enzyme is produced by cultivating a Hase producer microorganism and a DCase producer microorganism together.

14. A composite immobilized enzyme preparation comprising a hydantoinase and a decarbamylase which are derived from the same or different microorganisms, an antioxidant, manganese ion and a support directly carrying said enzymes, enzymatic activities of the hydantoinase and the decarbamylase directly carried by the support being almost equal at a reaction pH of between 6.5 to 8.0.

## Patentansprüche

1. Verfahren zur Herstellung einer D-Aminosäure aus dem entsprechenden DL-5-substituierten Hydantoin durch ein Einschrittverfahren umfassend die Verwendung einer immobilisierten Enzymzusammensetzung bei einem pH im neutralen Bereich, diese immobilisierte Enzymzusammensetzung wird erhalten durch gleichzeitiges Immobilisieren einer Mischung einer Hydantoinase, die ihren optimalen pH im alkalischen Bereich aufweist (im folgenden abgekürzt als Hase) und einer D-N-Carbamyl-αaminosäureamidohydrolase, deren optimaler pH im neutralen Bereich liegt (im folgenden abgekürzt als DCase), direkt an einen immobilisierenden Träger.

2. Verfahren zur Herstellung einer D-Aminosäure gemäß Anspruch 1, wobei eine Enzymzusammensetzung zur Immobilisierung verwendet wird, hergestellt durch getrennten Produzieren der Hase und der DCase und anschließendem Vermischen dieser.

3. Verfahren zur Herstellung einer D-Aminosäure gemäß Anspruch 1 oder 2, wobei die zu verwendende Hase eine ist, die von einem Mikroorganismus der Gattung Bacillus stammt.

4. Verfahren zur Herstellung einer D-Amihosäure gemäß Anspruch 1 oder 2, wobei die zu verwendende Hase eine ist, die von Bacillus sp. KNK108 (FERM P-6056) oder Bacillus sp. KNK245 (FERM BP-4863) stammt.

5. Verfahren zur Herstellung einer D-Aminosäure gemäß Anspruch 1 oder 2, wobei die zu verwendende Hase eine ist, die von einem rekombinanten E. coli stammt, insbesondere E. coli HB101pTH104 (FERM BP-4864).

6. Verfahren zur Herstellung einer D-Aminosäure gemäß Anspruch 1 oder 2, wobei die zu verwendende DCase eine ist, die von Agrobacterium sp. KNK712 (FERM BP-1900), Pseudomonas sp. KNK003A (FERM BP-3181), Pseudomonas sp. KNK505 (FERM BP-3182), E. coli JM109pAD108 (FERM BP-3184), E. coli JM109pPD304 (FERM BP-3183) stammt.

7. Verfahren zur Herstellung einer D-Aminosäure gemäß einem der Ansprüche 1 bis 4 oder 6, wobei die DCase eine ist, die von einem rekombinanten Mikroorganismus stammt, ausgewählt aus der Gruppe bestehend aus E. coli JM109pAD402 (FERM BP-3912), E. coli JM109pAD404 (FERM BP-3913), E. coli JM109pAD406 (FERM BP-3914), E. coli JM109pAD416 (PERM BP-3915), E. coli JM109pAD429 (FERM BP-4035, E. coli JM109pAD421, E. coli JM109pAD422, E. coli JM109pAD423, E. coli JM109pAD424 (FERM BP-4034), E. coli JM109pAD426, E. coli JM109pAD427, E. coli JM109pAD451, E. coli JM109pAD455 (FERM BP-4036) oder E. coli JM109pAD4553 (FERM BP-4368), wobei die Stabilität verbessert ist durch Aminosäureaustausch in der DCase, die von Agrobacterium sp. KNK712 (FERM BP-1900) stammt.

8. Verfahren zur Herstellung einer D-Aminosäure gemäß Anspruch 1, wobei eine zur Immobilisierung verwendete Enzymzusammensetzung hergestellt wird durch gleichzeitiges Produzieren von Hase und DCase.

9. Verfahren zur Herstellung einer D-Aminosäure gemäß Anspruch 8, wobei ein Mikroorganismus, der gleichzeitig Hase und die DCase produziert, ist: Agrobacterium sp. KNK-712 (PERM BP-1900), Rhizobium sp. KNK1415 (FERM BP-4419) oder Pseudomonas sp. KNK003A (FERM BP-3181).

10. Verfahren zur Herstellung einer D-Aminosäure gemäß Anspruch 8, wobei die Enzymzusammensetzung hergestellt wird durch Kultivieren eines Wirts enthaltend ein Plasmid, hergestellt durch Ligieren eines Hase-Gens und eines DCase-Gens in gleichen Vektor.

11. Verfahren zur Herstellung einer D-Aminosäure gemäß Anspruch 10, wobei der Wirt enthaltend ein Plasmid, hergestellt durch Ligieren des Hase-Gens und des DCase-Gens in einen gleichen Vektor ist: E. coli HB101pPHD301 (FERM BP-4866) oder E. coli JM109pAHD101.

12. Verfahren zur Herstellung einer D-Aminosäure gemäß Anspruch 8, wobei die Enzymzusammensetzung hergestellt wird durch Ligieren des Hase-Gens und des DCase-Gens in verschiedene Vektoren mit verschiedenen Replikationsmodi, Einfügen dieser Vektoren in den gleichen Wirt und Kultivieren des Wirts.

13. Verfahren zur Herstellung einer D-Aminosäure gemäß Anspruch 8, wobei die Enzymzusammensetzung hergestellt wird durch ein Miteinanderkultivieren eines Hase herstellenden Mikroorganismus und eines DCase herstellenden Mikroorganismus.

14. Immobilisierte Enzymzusammensetzungspräparation umfassend eine Hydantoinase und eine Decarbamylase, die von gleichen oder verschiedenen Mikroorganismen stammen, ein Antioxidans, Manganion und einen Träger, der direkt diese Enzyme trägt, die enzymatischen Aktivitäten der Hydantoinase und der Decarbamylase, die direkt auf den Träger aufgebracht sind, sind fast identisch bei einem Reaktions-pH zwischen 6,5 bis 8,0.

## Revendications

1. Procédé pour la production d'un acide aminé D à partir de l'hydantoïne DL 5-substituée correspondante par une réaction en une étape qui comprend le fait d'utiliser une enzyme composite immobilisée à un pH égal à environ la neutralité, ladite enzyme composite immobilisée étant obtenue par immobilisation simultanée d'un mélange d'une hydantoïnase ayant son pH optimal dans un intervalle alcalin (abrégé ci-dessous en Hase) et d'une acide D-N-carbamyl-α-aminé amidohydrolase ayant son pH optimal à environ la neutralité (abrégé ci-dessous en DCase) directement sur un support immobilisant.

2. Procédé pour la production d'un acide aminé D selon la revendication 1, dans lequel une enzyme composite à utiliser pour l'immobilisation est préparée en produisant la Hase et la DCase séparément, puis en les mélangeant.

3. Procédé pour la production d'un acide aminé D selon la revendication 1 ou 2, dans lequel la Hase à utiliser est celle dérivée d'un micro-organisme appartenant au genre Bacillus.

4. Procédé pour la production d'un acide aminé D selon la revendication 1 ou 2, dans lequel la Hase à utiliser est celle dérivée de Bacillus sp. KNK108 (FERM P-6056) ou de Bacillus sp. KNK245 (FERM BP-4863).

5. Procédé pour la production d'un acide aminé D selon la revendication 1 ou 2, dans lequel la Hase à utiliser est celle dérivée d'une E. coli recombinée, en particulier d'E. coli HB101pTH104 (FERM BP-4864).

6. Procédé pour la production d'un acide aminé D selon la revendication 1 ou 2, dans lequel la DCase à utiliser est celle dérivée d'Agrobacterium sp. KNK172 (FERM BP-1900), Pseudomonas sp. KNK003A (FERM BP-3181), de Pseudomonas sp. KNK505 (FERM BP-3182), d'E. coli JM109pAD108 (FERM BP-3184), d'E. coli JM109pPD304 (FERM BP-3183).

7. Procédé pour la production d'un acide aminé D selon l'une quelconque des revendications 1-4 ou 6, dans laquelle ladite DCase est celle dérivée d'un micro-organisme recombiné choisi dans le groupe consistant en *E. coli* JM109pAD402 (FERM BP-3912), *E. coli* JM109pAD404 (FERM BP-3913), *E. coli* JM109pAD406 (FERM BP-3914), *E. coli* JM109pAD416 (FERM BP-3915), *E. coli* JM109pAD429 (FERM BP-4035), *E. coli* JM109pAD421, *E. coli* JM109pAD422, *E. coli* JM109pAD423, *E. coli* JM109pAD424 (FERM BP-4034), *E. coli* JM109pAD426, *E. coli* JM109pAD427, *E. coli* JM109pAD451, *E. coli* JM109pAD455 (FERM BP-4036), ou *E. coli* JM109pAD4553 (FERM BP-4368), dont la stabilité est augmentée par une substitution d'acides aminés de la DCase dérivée d'*Agrobacterium* sp. KNK712 (FERM BP-1900).

8. Procédé pour la production d'un acide aminé D selon la revendication 1, dans lequel on prépare une enzyme composite utilisée pour l'immobilisation en produisant simultanément la Hase et la DCase.

9. Procédé pour la production d'un acide aminé D selon la revendication 8, dans lequel un micro-organisme qui produit la Hase et la DCase simultanément est Agrobacterium sp. KNK-172 (FERM BP-1900), Rhizobium sp. KNK1415 (FERM BP-4419) ou Pseudomonas sp. KNK003A (FERM BP-3181).

10. Procédé pour la production d'un acide aminé D selon la revendication 8, dans lequel l'enzyme composite est produite en cultivant un hôte contenant un plasmide préparé en ligaturant un gène de Hase et un gène de DCase au même vecteur.

11. Procédé pour la production d'un acide aminé D selon la revendication 10, dans lequel l'hôte contenant un plasmide préparé en ligaturant le gène de Hase et le gène de DCase au même vecteur est E. coli HB101pPHD301 (FERM BP-4866) ou E. coli JM109pAHD101.

12. Procédé pour la production d'un acide aminé D selon la revendication 8, dans lequel l'enzyme composite est produite en ligaturant le gène de Hase et le gène de DCase à des vecteurs différents ayant des modes de réplication différents, en introduisant ces vecteurs dans le même hôte et en cultivant l'hôte.

13. Procédé pour la production d'un acide aminé D selon la revendication 8, dans lequel l'enzyme composite est produite en cultivant ensemble un micro-organisme producteur de Hase et un micro-organisme producteur de DCase.

14. Préparation d'enzyme composite immobilisée comprenant une hydantoïnase et une décarbamylase qui sont dérivées du même micro-organisme ou de micro-organismes différents, un antioxydant, d'ion manganèse et un support portant directement lesdites enzymes, les activités enzymatiques de l'hydantoïnase et de la décarbamylase directement portées par le support étant presque égales à un pH de réaction entre 6,5 et 8,0.
